(19) Europäisches Patentamt
European Patent Office
Office européen des brevets

(11) Veröffentlichungsnummer: **0 331 919 B1**

(12) **EUROPÄISCHE PATENTSCHRIFT**

(45) Veröffentlichungstag der Patentschrift: **04.08.93**

(51) Int. Cl.[5]: **C07D 333/36**, C07D 333/48

(21) Anmeldenummer: **89102212.1**

(22) Anmeldetag: **09.02.89**

(54) **Verfahren zur Herstellung von Thiophenderivaten sowie Dihydrothiophen-1-oxide.**

(30) Priorität: **16.02.88 DE 3804794**

(43) Veröffentlichungstag der Anmeldung:
**13.09.89 Patentblatt 89/37**

(45) Bekanntmachung des Hinweises auf die Patenterteilung:
**04.08.93 Patentblatt 93/31**

(84) Benannte Vertragsstaaten:
**AT BE CH DE ES FR GB IT LI NL SE**

(56) Entgegenhaltungen:
**WO-A-87/02220**
**DE-A- 2 700 215**
**GB-A- 2 194 530**

**BULLETIN OF THE CHEMICAL SOCIETY OF JAPAN. Band 41, Nr. 9, 1968, Seiten 2086-2095, Tokyo; Takao Tukaya et al.:"Preparation and Oxidation of Dihydrothiophenes"**

**HELVETICA CHIMICA ACTA. Band 57, Fasc. 8, Nr.269270, 1974, Seiten 2487-2492; A P Stoll et al.: "Asymmetrische homogene Hydrierung eines Thiophenderivatives"**

**M. FIESER et al.:" REAGENTS FOR ORGANIC SYNTHESIS" Band 4, John Wiley & Sons, New York. Page 8, para 2**

**W. THEILHEIMER: "SYNTHETIC METHODS OF ORGANIC CHEMISTRY" Band 17, 1963, S Karger, Basel. Page 233, Nr 575**

(73) Patentinhaber: **HOECHST AKTIENGESELLSCHAFT**

**W-6230 Frankfurt am Main 80(DE)**

(72) Erfinder: **Rippel, Robert, Dr.**
**Frankfurter Strasse 66**
**W-6238 Hofheim am Taunus(DE)**
Erfinder: **Flemming, Hans-Wolfram, Flemming, Dr.**
**An den Tannen 3**
**W-6390 Usingen(DE)**
Erfinder: **Rukwied, Manfred, Dr.**
**Fasanenstrasse 28**
**W-6233 Kelkheim (Taunus)(DE)**

**EP 0 331 919 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, daß man Thiophenderivate durch Dehydrierung von Dihydrothiophenen herstellung kann, wobei als Dehydrierungsmittel z.B. Chloride und Bromide der Schwefel-oder Phosphorsäure, Chlor, Brom, N-Halogenverbindungen, Tetrachlorchinon (Chloranil), Nitrosobenzol, Jodosobenzol, Selen oder Schwefel verwendet werden. Für bestimmte Fälle ist als Dehydrierungsmittel auch Wasserstoffperoxid $H_2O_2$ bereits beschrieben worden; vgl. z.B. A.P. Stoll und R. Süess, Helvetica Chimica Acta Vol. 57, Fasc. 8 (1974) Nr. 269-270, S. 2487-2492. Nach dieser Literaturstelle wird 4-Ethoxycarbonyl-3-hydroxy-2-phenyl-dihydrothiophen mit $H_2O_2$ in ethanolischer Lösung bei 60 - 65 °C zu dem entsprechenden Thiophenderivat dehydriert; die Reaktion verläuft nach folgender Formelgleichung:

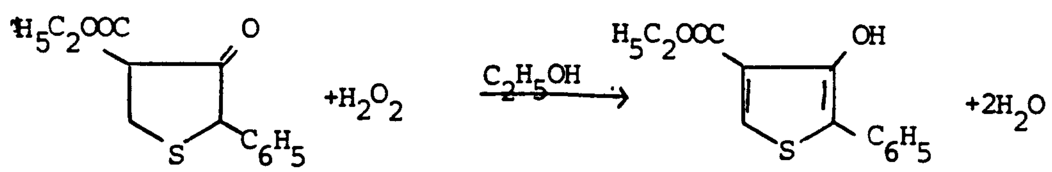


Als Ausbeute ist für diese Reaktion 90 % angegeben.

Aus anderen Dihydrothiophenen - nämlich solchen, die als Substituenten Elektronen liefernde Gruppen wie z.B. die Aminogruppe bzw. eine substituierte Aminogruppe tragen - entstehen mit $H_2O_2$ in Eisessig nicht die entsprechenden Thiophenderivate, sondern die Dihydrothiophen-Sulfone; vgl. T.Takaya et al., Bull. Chem. Soc. Japan, Vol. 41, S. 2086-2095 (1968). Ein in dieser Literaturstelle speziell beschriebenes Beispiel ist die Umsetzung von 3-Ethoxycarbonylamino-4-ethoxycarbonyl-2,5-dihydrothiophen mit $H_2O_2$/Eisessig:

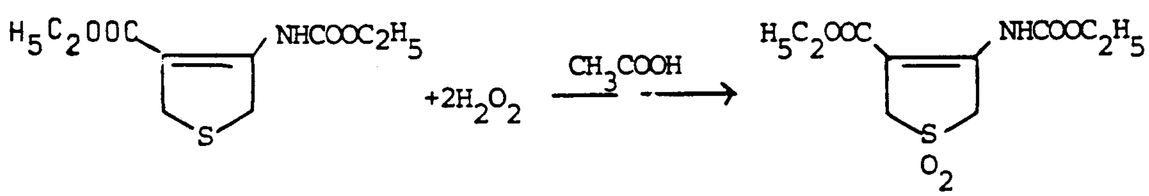


Sulfon

Es wurde nun gefunden, daß man auch solche Dihydrothiophene, welche die Aminogruppe bzw. eine substituierte Aminogruppe als Substituenten tragen, mit $H_2O_2$ zu den entsprechenden Thiophenderivaten dehydrieren kann, wenn man die Dehydrierung in 2 Stufen durchführt, nämlich

zuerst Umsetzung der Dihydrothiophene mit $H_2O_2$ in neutralem Medium, wobei die Dihydrothiophen-1(=S)oxide entstehen, und danach Versetzen der Dihydrothiophen-1-oxide - ohne oder auch nach deren Isolierung - mit einer Säure, wobei die Umlagerung zu den entsprechenden Thiophenderivaten erfolgt.

Erfindungsgegenstand ist daher ein Verfahren zur Herstellung von Thiophenderivaten der Formel I

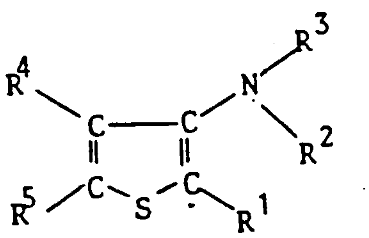

I

worin $R^1$ - $R^5$ unabhängig voneinander Wasserstoff (H) oder

$R^1$, $R^4$ und $R^5$: $C_1$-$C_4$-Alkyl,

$C_5$-$C_6$-Cycloalkyl,
$CH_2$-$C_6H_5$,
$C_6H_5$,
$COOCH_3$,
$COOC_2H_5$,
$CONH_2$,
$COCH_3$,
$COC_2H_5$ oder
CN;

$R^2$ und $R^3$: $C_1$-$C_4$-Alkyl, gegebenenfalls substituiert durch $COOCH_3$ oder $COOC_2H_5$,
$C_5$-$C_6$-Cycloalkyl,
$(CH_2)_{1-3}$-$C_6H_5$,
$C_6H_5$, gegebenenfalls substituiert durch OH, $OCH_3$, $OC_2H_5$, F, Cl oder Br,
$CO(C_1$-$C_5$-Alkyl), wobei der Alkylrest durch OH, F, Cl, Br, ($C_1$-$C_4$)-Alkyloxy, $NH_2$ oder ($C_1$-$C_8$)-Alkylaminogruppen substituiert sein kann,

und einer der Reste $R^2$ oder $R^3$ zusätzlich noch $COOCH_3$ oder $COOC_2H_5$ sein kann,
oder $R^2$ und $R^3$ zusammen mit dem N-Atom, an das sie gebunden sind,
einen Pyrrolidino-, Piperidino-, Morpholino-, Piperazino- oder Homopiperazino-Ring bilden können, bedeuten,
durch Dehydrierung von Dihydrothiophenen der Formeln IIa und/oder IIb

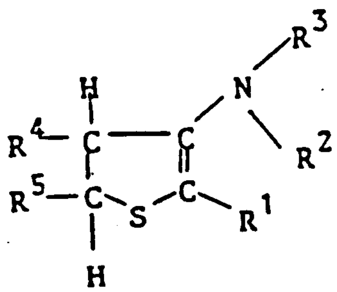
IIa

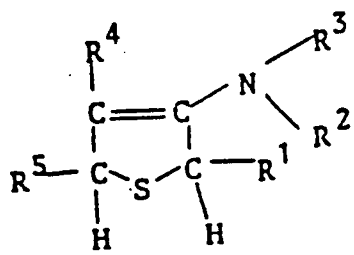
IIb

worin die Reste $R^1$ - $R^5$ die gleiche Bedeutung wie in Formel I besitzen,
das dadurch gekennzeichnet ist, daß man die Dehydrierung mit $H_2O_2$ und in 2 Stufen durchführt, indem man

a) die Dihydrothiophene der Formeln IIa und/oder IIb mit einer mindestens etwa äquimolaren Menge $H_2O_2$ in einem neutralen Lösungs- oder Verdünnungsmittel in Abwesenheit von Säure(n) umsetzt, wobei die 1-Oxide der Ausgangs-dihydrothiophene entstehen, und

b) die in Stufe a) gebildeten Dihydrothiophen-1-oxide ohne oder auch nach ihrer Isolierung mit einer Säure versetzt, wobei sich die Dihydrothiophen-1-oxide zu den Thiophenderivaten der Formel I umlagern.

Formelmäßig läßt sich das Verfahren wie folgt darstellen:

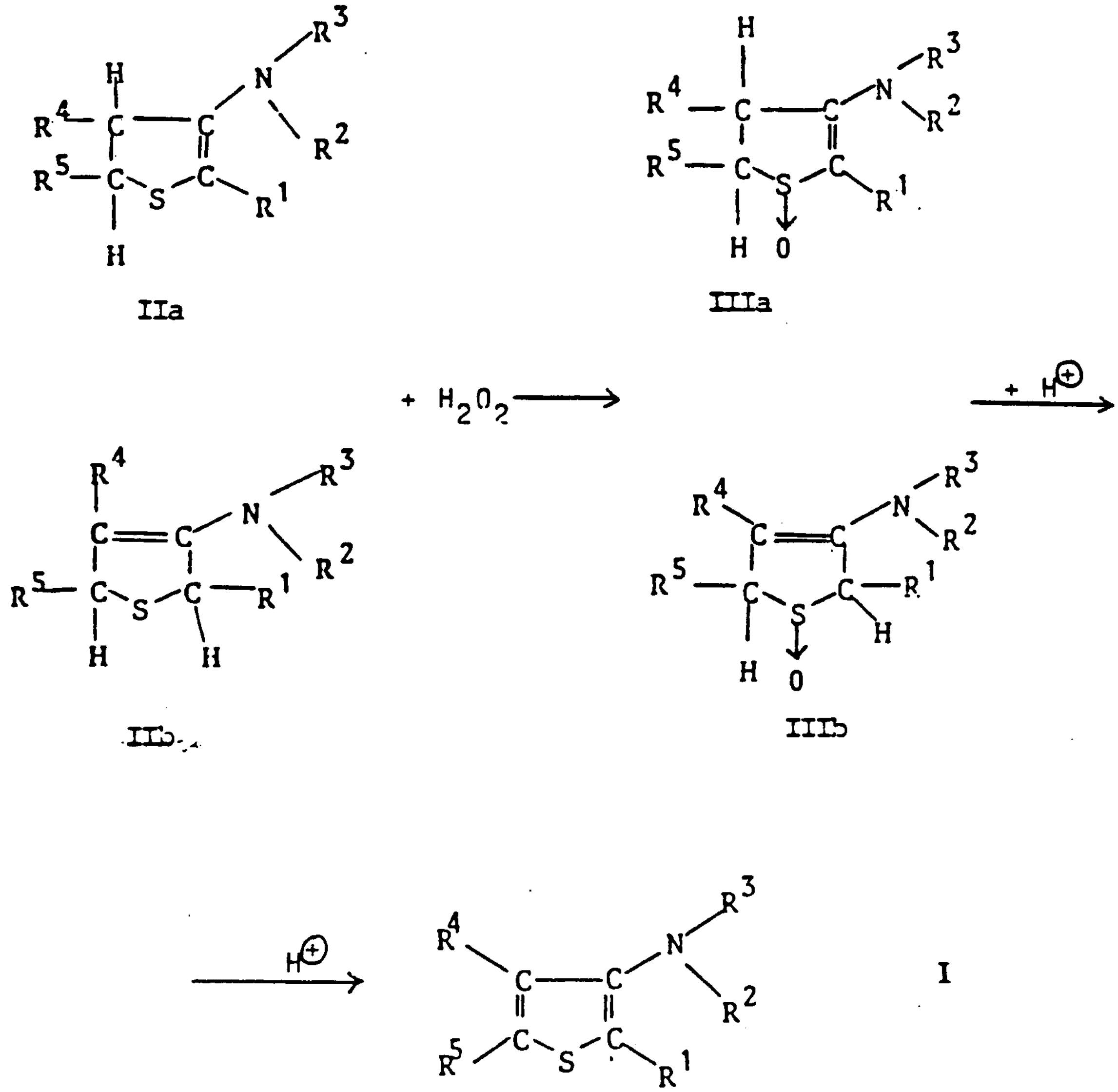


IIa IIIa

IIb IIIb

I

Auch die beiden einzelnen Verfahrensstufen a) und b) sowie die in Stufe a) gebildeten Dihydrothiophen-1-oxide (der Formeln IIIa/IIIb) sind Erfindungsgegenstand.

Es war nicht zu erwarten und ist daher außerordentlich überraschend, daß die von aminogruppen-substituierten Dihydrothiophenen und $H_2O_2$ ausgehende Reaktion von T.Takaya et al. (a.a.O.) durch die erfindungsgemäße Abänderung in eine ganz andere Richtung - nämlich auf die Bildung der entsprechenden Thiophenderivate zu (anstelle der Dihydrothiophen-Sulfone) - gelenkt werden kann. Maßgebend für diesen andersartigen Verlauf ist ganz offensichtlich, daß zu Beginn der Reaktion keine Säure zugegen ist.

Das Verfahren liefert hohe Ausbeuten und Reinheiten der Produkte und ist außerordentlich umwelt- und entsorgungsfreundlich. Bisher konnte man aminogruppen-substituierte Dihydrothiophene nur mit weniger umwelt- und entsorgungsfreundlichen Dehydrierungsmitteln in die entsprechenden Thiophenderivate umwandeln; die Möglichkeit, diese Umwandlung nun auch mit $H_2O_2$ - aus welchem außer Wasser keinerlei Nebenprodukte entstehen - durchführen zu können, stellt einen außerordentlichen Vorteil und Fortschritt dar.

Die Substituenten $R^1$ bis $R^5$ in den vorerwähnten Formeln I, IIa/IIb und IIIa/IIIb können - unabhängig voneinander - H oder die gleiche Bedentung wie in Formel I besitzen.

Die Ausgangs-Dihydrothiophene der Formeln IIa und IIb sind entweder literaturbekannt oder können in Analogie zu bekannten Verfahren hergestellt werden. Bekannte Verfahren sind z.B. angegeben in der vorerwähnten Literaturstelle von T.Takaya et al. (a.a.O.) - vgl. insbesondere S. 2090 - und in DE-C 1 643

325. Ein zusammenfassender neuerer Überblick ist enthalten in dem Buch von Weissberger, The Chemistry of Heterocyclic Compounds, A Series of Monographs, Arnold Weissberger and Edward C. Taylor, Vol. 44/Part 21 "Thiophene and Its Derivatives" Edited by S. Gronowitz (1986).

Die Ausgangs-Dihydrothiophene werden in Stufe a) des erfindungsgemäßen Verfahrens mit einer mindestens etwa äquimolaren Menge - vorzugsweise mit einer etwa 1- bis 2-molaren Menge - $H_2O_2$ in einem neutralen Lösungs- oder Verdünnungsmittel in Abwesenheit von Säure(n) umgesetzt.

Als neutrale Lösungs- oder Verdünnungsmittel kommen Wasser und/oder neutrale organische Lösungsmittel in Frage. Als neutrale organische Lösungsmittel sind in beispielhafter Weise zu nennen:
Alkohole wie z.B. Methanol, Ethanol, 1-Propanol, Isopropanol, 1-Butanol, 2-Butanol, tert.-Butanol, 2-Methylpropanol;
aliphatische und cycloaliphatische Kohlenwasserstoffe wie z.B. Pentan, 2-Methylbutan, Hexan, 2.2-Dimethylbutan, 2.3-Dimethylbutan, 2-Methylpentan, 3-Methylpentan, Heptan, Cyclohexan, Methylcyclohexan, 1.2-Dimethylcyclohexan, 1.3-Dimethylcyclohexan, 1.4-Dimethylcyclohexan;
aromatische Kohlenwasserstoffe wie z.B. Toluol, Xylole, Isopropylbenzol;
aliphatische und aromatische Halogenkohlenwasserstoff wie z.B. Tetrachlorethylen, 1.1.2.2- und 1.1.1.2-Tetrachlorethan, Methylenchlorid, Dichlorpropan, Tetrachlorkohlenstoff, 1.12-Trichlor-1.22-trifluorethan, Trichlorfluormethan, 1.2-Di-chlorethan, 1.1-Dichlorethan, Chlorbenzol;
Ether wie z.B. Diethylether, Di-n-butylether, Diisopropylether, Diisoamylether, Methyl-tert.-butylether, Ethylenglykoldimethylether, Tetrahydrofuran, Dioxan, Anisol;
Ester wie z.B. Methylacetat, Ethylacetat, Butylacetat;
Säureamide wie z.B. Dimethylformamid, Dimethylacetamid.

Besonders bevorzugte Lösungs- oder Verdünnungsmittel sind Wasser und $C_1$-$C_4$-Alkanole (insbesondere Ethanol und Isopropanol), alleine oder in Mischung miteinander.

Die Reaktionstemperatur in Stufe a) kann in einem ziemlich weiten Bereich schwanken. Im allgemeinen liegt die Reaktionstemperatur hier zwischen etwa -20 und etwa +100°C, vorzugsweise zwischen etwa -15 und etwa +70°C.

Zur Durchführung der Reaktion werden zunächst die entsprechenden Dihydrothiophene der Formeln IIa und/oder IIb in dem entsprechenden neutralen Lösungs- oder Verdünnungsmittel gelöst oder suspendiert. Dazu wird dann normalerweise die entsprechende Menge $H_2O_2$ -zweckmäßig ebenfalls in Mischung mit einem oder mehreren der vorher genannten Lösungs- oder Verdünnungsmittel - getropft und so lange bei der Reaktionstemperatur gehalten, bis kein Ausgangsmaterial mehr nachgewiesen werden kann; der Nachweis erfolgt auf übliche Weise, zweckmäßig durch Probenentnahme und Dünnschichtchromatogramm.

Die gebildeten Dihydrothiophen-1-oxide der Formeln IIIa bzw. IIIb können entweder durch Abkühlen und Absaugen oder durch Einengen der Lösung isoliert werden. Sie können gegebenenfalls als Diastereomerengemische auftreten, die chromatographisch getrennt werden können. Die Dihydrothiophen-1-oxide der Formeln IIIa und IIIb sind neue Verbindungen.

Bei der Umlagerung gemäß Stufe b) des erfindungsgemäßen Verfahrens ergeben die Dihydrothiophen-1-oxide der Formeln IIa und IIb die gleichen Thiophenderivate. Für diese Umlagerung können die isolierten Dihydrothiophen-1-oxide wieder in einem der vorerwähnten Lösungs- oder Verdünnungsmittel gelöst oder suspendiert werden, oder es kann auch die in Stufe a) erhaltene Raktionsmischung gleich als solche weiter verarbeitet werden.

In die Lösung oder Suspension wird eine Säure zugetropft bzw. gasförmig (HCl, HBr!) eingeleitet. Je nach den Ausgangsverbindungen sind dabei katalytische oder auch höhere Säuremengen zweckmäßig bzw. notwendig.

Als Säuren können anorganische und/oder organische Säuren verwendet werden. Bevorzugt sind wäßrige, alkoholische oder Etherische Lösungen von Chlor- oder Bromwasserstoff oder von Schwefelsäure.

Die mögliche Reaktionstemperatur umfaßt den Bereich zwischen dem Erstarrungspunkt und der Siedetemperatur des verwendeten Lösungs- oder Verdünnungsmittels; bevorzugt ist hier Raumtemperatur.

Die Aufarbeitung des Ansatzes erfolgt in üblicher Weise. Aus den gegebenenfalls entstandenen Aminothiophensalzen können die freien Aminothiophene in bekannter Weise erhalten werden.

Die neuen Dihydrothiophen-1-oxide sowie auch die durch deren Umlagerung nach dem erfindungsgemäßen Verfahren entstehenden bekannten Thiophenderivate sind wertvolle Ausgangs- bzw. Zwischenprodukte für die Herstellung insbesondere von Pflanzenschutzmitteln und Pharmazeutika. Als Pharmazeutika sind hier z.B. die in der vorerwähnten DE-C-1 643 325 beschriebenen substituierten 3-Aminoacylaminothiophene der nachstehenden allgemeinen Formel IV zu nennen, welche sich vor allem als Lokalanästhetika eignen:

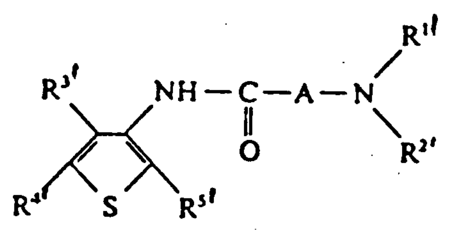

(IV)

worin
$R^{1'}$ = H oder organischer Rest,
$R^{2'}$ = organischer Rest
oder $R^{1'}$ und $R^{2'}$ auch - zusammen mit dem N-Atom, an das sie gebunden sind - einen Ring bilden können,
$R^{3'}$ - $R^{5'}$ = H oder organische Reste, und
A = $C_1$-$C_4$-Alkylengruppe.

Die Verbindungen werden z.B. nach Variante b) des Verfahrens der DE-C wie folgt hergestellt:

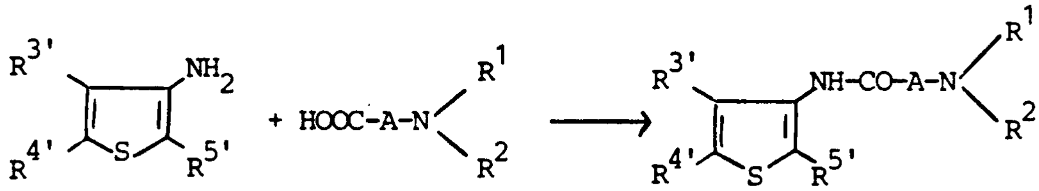

(V) (VI) (IV)

Die Ausgangsprodukte (V) können nach dem erfindungsgemäßen Verfahren über die neuen Zwischenprodukte IIIa/IIIb vorteilhafter und umweltfreundlicher als bisher dargestellt werden.

Die nachfolgenden Beispiele dienen zur Erläuterung der Erfindung, ohne diese jedoch einzuschränken. Die angegebenen Schmelz- und Zersetzungspunkte sind nicht korrigiert und abhängig von eventuell auftretenden Diastereomerengemischen.

**Beispiel 1**

**a) 3-Amino-4-methoxycarbonyl-2,5-dihydrothiophen-1-oxid**

3,2 g (0,02 Mol) 3-Amino-4-methoxycarbonyl-2,5-dihydrothiophen werden in 30 ml Isopropanol suspendiert. Unter Kühlung werden 2.2 ml 35 %iges Wasserstoffperoxid zugegeben. Anschließend wird bei 50 °C Innentemperatur solange gerührt bis im Dünnschichtchromatogramm kein Ausgangsmaterial mehr nachweisbar ist (ca. 5 Stunden). Dann wird im Eisbad gekühlt, die ausgefallenen Kristalle abgesaugt, mit eiskaltem Isopropanol und mit Diethylether gewaschen. Ausbeute: 3,5 g ( 91 % d.Th.). Aus Methanol umkristallisiert schmelzen die Kristalle bei 137 - 140 °C.

In analoger Weise wurde eine Reihe anderer Dihydrothiophen-1-oxide hergestellt; die Details sind aus Tabelle 1 ersichtlich.

Tabelle 1

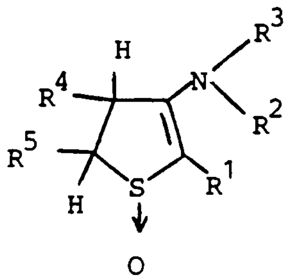

bzw.

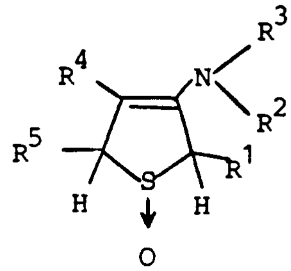

| No. | $R^1$ | $R^2$ | $R^3$ | $R^4$ | $R^5$ | Fp.(°C) |
|---|---|---|---|---|---|---|
| 1a | H | H | H | $COOCH_3$ | H | 137-140 |
| 1b | $COOCH_3$ | H | H | $CH_3$ | H | 212-218 |
| 1c | H | H | H | CN | H | 165-167 |
| 1d | H | $-(CH_2)_2C_6H_5$ | H | $COOCH_3$ | H | Öl |
| 1e | $COOCH_3$ | $-COCH_3$ | H | $CH_3$ | H | 93- 98 |
| 1f | H | $-COCH_3$ | H | $-COOCH_3$ | H | 119-122 |
| 1g | H | $-COOC_2H_5$ | H | $-COOCH_3$ | H | Öl |
| 1h | $COOCH_3$ | $-COOC_2H_5$ | H | $CH_3$ | H | 132-135 |
| 1i | $COOCH_3$ | $-C_6H_4-4-OCH_3$ | H | $CH_3$ | H | 158-165 |
| 1k | $COOCH_3$ | $-CH_3$ | H | $CH_3$ | H | Öl |
| 1l | H | $CH_3$ | $C_6H_5$ | CN | H | Öl |
| 1m | H | $CH_3$ | $CH_3$ | CN | H | 88- 93 |
| 1n | $CH_3$ | H | H | $COOCH_3$ | H | 131-138 |
| 1o | $COCH_3$ | H | H | $CH_3$ | H | 171-175 |
| 1p | H | $COCH_3$ | H | CN | H | Öl |
| 1q | $COOCH_3$ | $CH_2-COOC_2H_5$ | H | $CH_3$ | H | 93- 98 |
| 1r | $COOCH_3$ | $CH_2-COOCH_3$ | H | $CH_3$ | H | Öl |
| 1s | $COOCH_3$ | $OC-CH(CH_3)-NHC_3H_7$ | H | $CH_3$ | H | Öl |

**Beispiel 2**

**3-Amino-4-methoxycarbonyl-thiophen**

3,8 g (0.02 Mol) 3-Amino-4-methoxycarbonyl-2,5-dihydrothiophen-1-oxid werden in 20 ml Isopropanol suspendiert. Unter Eiskühlung werden 5 ml einer 5 n Lösung von Chlorwasserstoff in Isopropanol zugetropft und anschließend ca. 1 Stunde bei 60 °C Badtemperatur gerührt. Wenn das Dünnschichtchromatogramm vollständige Umsetzung zeigt, wird die Suspension vollständig einrotiert, der Rückstand mit wenig Essigester verrieben, abgesaugt und getrocknet. Ausbeute: 2,8 g (89 % d.Th.); Fp. 194-201 °C).

In analoger Weise wurden die aus Tabelle 2 ersichtlichen Thiophenderivate hergestellt.

**Tabelle 2**

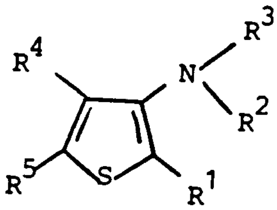

| No. | $R^1$ | $R^2$ | $R^3$ | $R^4$ | $R^5$ | Fp.(°C) |
|---|---|---|---|---|---|---|
| 2a | H | H | H | $COOCH_3$ | H | 194-201 (HCl) |
| 2b | $COOCH_3$ | H | H | $CH_3$ | H | 128-131 (HCl) |
| 2c | H | H | H | CN | H | 214-216 (HCl) |
| 2d | H | $-(CH_2)_3C_6H_5$ | H | $COOCH_3$ | H | 145-148 (HCl) |

Tabelle 2 (Forts.)

| No. | $R^1$ | $R^2$ | $R^3$ | $R^4$ | $R^5$ | Fp.(°C) |
|---|---|---|---|---|---|---|
| 2e | $COOCH_3$ | $COCH_3$ | H | $CH_3$ | H | 117 |
| 2f | H | $COCH_3$ | H | $COOCH_3$ | H | 58- 60 |
| 2g | H | $-COOC_2H_5$ | H | $COOCH_3$ | H | 49- 52 |
| 2h | $COOCH_3$ | $-COOC_2H_5$ | H | $CH_3$ | H | 80- 83 |
| 2i | $COOCH_3$ | $-C_6H_4-4-OCH_3$ | H | $CH_3$ | H | 97-100 |
| 2k | $COOCH_3$ | $CH_3$ | H | $CH_3$ | H | 151-154 (HCl) |
| 2l | H | $C_6H_5$ | $CH_3$ | CN | H | 52- 53 |
| 2m | H | $CH_3$ | $CH_3$ | CN | H | 142-144 (HCl) |
| 2n | $CH_3$ | H | H | $COOCH_3$ | H | 154-159 (HCl) |
| 2o | $COCH_3$ | H | H | $CH_3$ | H | 161-162 (HCl) |
| 2p | H | $-COCH_3$ | H | CN | H | 162-167 |
| 2q | $COOCH_3$ | $-CH_2COOC_2H_5$ | H | $CH_3$ | H | 48- 50 |
| 2r | $COOCH_3$ | $-CH_2-COOCH_3$ | H | $CH_3$ | H | 72- 74 |
| 2s | $COOCH_3$ | $OC-\overset{CH_3}{\overset{\vert}{C}}H-NHC_3H_7$ | H | $CH_3$ | H | 175-176 (HCl) |

**Beispiel 3**

**3-Amino-2-methoxycarbonyl-4-methylthiophen**

3,46 g (0.02 Mol) 3-Amino-2-carbomethoxy-4-methyl-4,5-dihydrothiophen werden in 25 ml Isopropanol gelöst und unter Eiskühlung 2,2 ml 35 %iges Wasserstoffperoxid zugetropft. Man läßt 30 Minuten bei Raumtemperatur nachrühren. Anschließend wird mit Eiswasser gekühlt und 4 ml 5 n isopropanolische Salzsäure zugetropft. Dann wird auf 60 °C erwärmt und ca. 90 Minuten bei dieser Temperatur gerührt. Zur Aufarbeitung wird das Isopropanol abdestilliert, der kristalline Rückstand in wenig Essigester ausgerührt, abgesaugt und getrocknet. Ausbeute an Hydrochlorid: 3,86 g (= 93 % d.Th.) vom Schmelzpunkt 128-130 °C.
Schmelzpunkt der freien Base: 85 °C.

In analoger Weise lassen sich auch die in Beispiel 2 beschriebenen Verbindungen im Eintopfverfahren herstellen.

**Patentansprüche**

**Patentansprüche für folgende Vertragsstaaten : DE, GB, FR, IT, NL, SE, LI, CH, BE, AT**

**1.** Verfahren zur Herstellung von Thiophenderivaten der Formel I

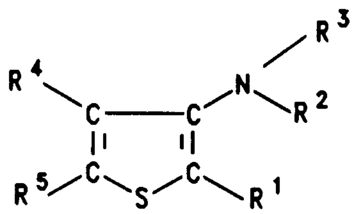

I

worin $R^1$ bis $R^5$ folgende Bedeutung besitzen:

$R^1$, $R^4$ und $R^5$: unabhängig voneinander H,
$C_1$-$C_4$-Alkyl,
$C_5$-$C_6$-Cycloalkyl,
$CH_2$-$C_6H_5$,
$C_6H_5$,
$COOCH_3$,
$COOC_2H_5$,
$CONH_2$,
$COCH_3$,
$COC_2H_5$ oder
CN;

$R^2$ und $R^3$: unabhängig voneinander H, $C_1$-$C_4$-Alkyl,
gegebenenfalls substituiert durch $COOCH_3$ oder $COOC_2H_5$,
$C_5$-$C_6$-Cycloalkyl,
$(CH_2)_{1-3}$-$C_6H_5$,
$C_6H_5$, gegebenenfalls substituiert durch OH, $OCH_3$, $OC_2H_5$, F, Cl oder Br,
CO($C_1$-$C_5$-Alkyl), wobei der Alkylrest durch OH, F, Cl, Br, ($C_1$-$C_4$)-Alkyloxy, $NH_2$ oder ($C_1$-$C_8$)-Alkylaminogruppen substituiert sein kann,

und einer der Reste $R^2$ oder $R^3$ zusätzlich noch $COOCH_3$ oder $COOC_2H_5$ sein kann, oder $R^2$ und $R^3$ zusammen mit dem N-Atom, an das sie gebunden sind, einen Pyrrolidino-, Piperidino-, Morpholino-, Piperazino- oder Homopiperazino-Ring bilden, durch Dehydrierung von Dihydrothiophenen der Formeln IIa und/oder IIb

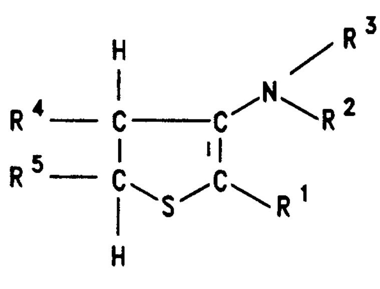

IIa

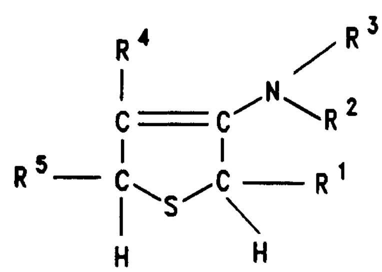

IIb

wobei die Reste $R^1$ bis $R^5$ die gleiche Bedeutung wie in Formel I besitzen, dadurch gekennzeichnet, daß man die Dehydrierung mit $H_2O_2$ und in zwei Stufen durchführt, indem man

a) die Dihydrothiophene der Formeln IIa und/oder IIb mit einer mindestens etwa äquimolaren Menge $H_2O_2$ in einem neutralen Lösungs- und Verdünnungsmittel in Abwesenheit von Säure(n) umsetzt, wobei die 1-Oxide der Ausgangs-Dihydrothiophene entstehen und

b) die in Stufe a) gebildeten Dihydrothiophen-1-oxide ohne oder auch nach ihrer Isolierung mit einer Säure versetzt, wobei sich die Dihydrothiophen-1-oxide in die Thiophenderivate der Formel I umlagern.

**2.** Verfahren zur Herstellung von Dihydrothiophen-1-oxiden der Formeln IIIa und IIIb

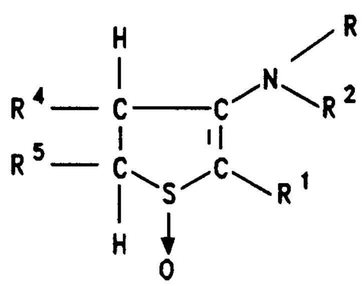

IIIa

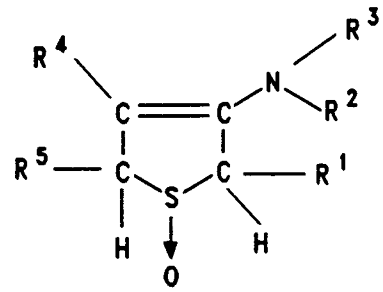

IIIb

worin $R^1$ bis $R^5$ unabhängig voneinander die in Anspruch 1 genannte Bedeutung haben, dadurch gekennzeichnet, daß man Dihydrothiophene der Formeln IIa bzw. IIb

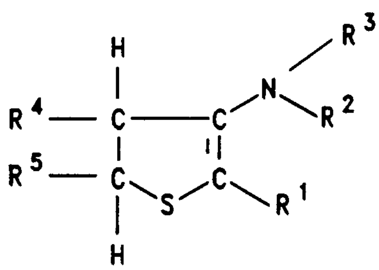

IIa

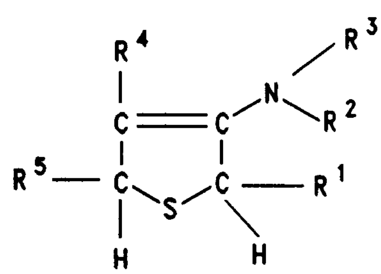

IIb

worin $R^1$ bis $R^5$ die gleiche Bedeutung wie in den Formeln IIIa und IIIb besitzen, mit einer mindestens etwa äquimolaren Menge $H_2O_2$ in einem neutralen Lösungs- oder Verdünnungsmittel in Abwesenheit von Säure(n) umsetzt.

**3.** Verfahren zur Herstellung von Thiophenderivaten der Formel I gemäß der Definition in Anspruch 1, dadurch gekennzeichnet, daß man Dihydrothiophen-1-oxide der Formeln IIIa und/oder IIIb

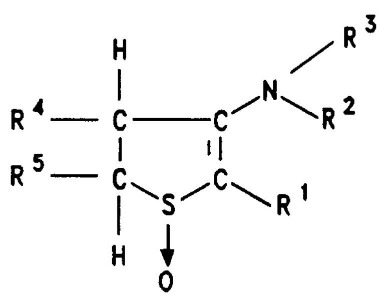

IIIa

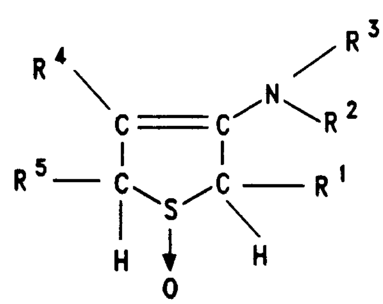

IIIb

worin $R^1$ bis $R^5$ unabhängig voneinander die in Anspruch 1 genannte Bedeutung haben, mit einer Säure versetzt, wobei sich die Dihydrothiophen-1-oxide zu den Thiophenderivaten der Formel I umlagern.

**4.** Verfahren nach einem oder mehreren der Ansprüche 1 bis 3, dadurch gekennzeichnet, daß man in Wasser oder einem neutralen organischen Lösungsmittel, vorzugsweise in Wasser und/oder einem $C_1$-$C_4$-Alkanol, als Lösungs- oder Verdünnungsmittel arbeitet.

**5.** Dihydrothiophen-1-oxide der Formeln IIIa und IIIb

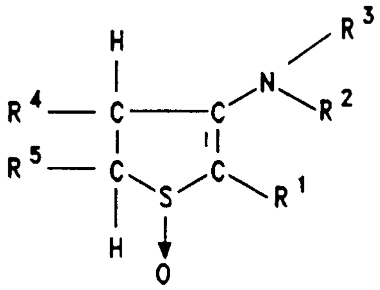

IIIa

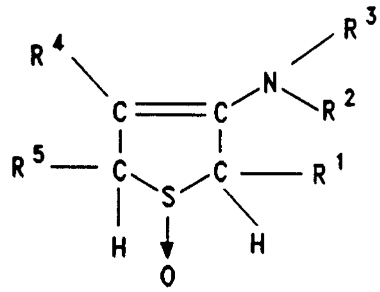

IIIb

worin $R^1$ bis $R^5$ die in Anspruch 1 genannte Bedeutung haben.

**Patentansprüche für folgenden Vertragsstaat : ES**

**1.** Verfahren zur Herstellung von Thiophenderivaten der Formel I

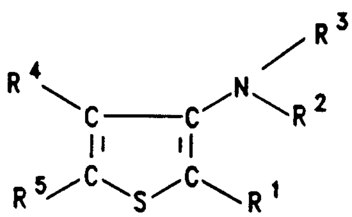

I

worin $R^1$ bis $R^5$ folgende Bedeutung besitzen:

$R^1$, $R^4$ und $R^5$: unabhängig voneinander H,
$C_1$-$C_4$-Alkyl,
$C_5$-$C_6$-Cycloalkyl,
$CH_2$-$C_6H_5$,
$C_6H_5$,
$COOCH_3$,
$COOC_2H_5$,
$CONH_2$,
$COCH_3$,
$COC_2H_5$ oder
CN;

$R^2$ und $R^3$: unabhängig voneinander H, $C_1$-$C_4$-Alkyl,
gegebenenfalls substituiert durch $COOCH_3$ oder $COOC_2H_5$,
$C_5$-$C_6$-Cycloalkyl,
$(CH_2)_{1-3}$-$C_6H_5$,
$C_6H_5$, gegebenenfalls substituiert durch OH, $OCH_3$,
$OC_2H_5$, F, Cl oder Br,
CO($C_1$-$C_5$-Alkyl), wobei der Alkylrest durch OH, F, Cl, Br, ($C_1$-$C_4$)-Alkyloxy, $NH_2$ oder ($C_1$-$C_8$)-Alkylaminogruppen substituiert sein kann,

und einer der Reste $R^2$ oder $R^3$ zusätzlich noch $COOCH_3$ oder $COOC_2H_5$ sein kann,
oder $R^2$ und $R^3$ zusammen mit dem N-Atom, an das sie gebunden sind,

einen Pyrrolidino-, Piperidino-, Morpholino-, Piperazino- oder Homopiperazino-Ring bilden, durch Dehydrierung von Dihydrothiophenen der Formeln IIa und/oder IIb

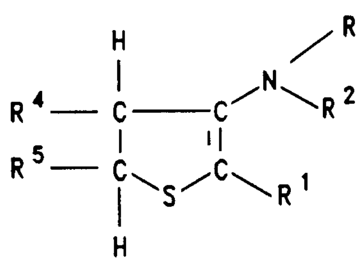

II a

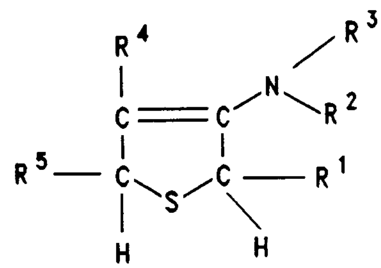

II b

wobei die Reste $R^1$ bis $R^5$ die gleiche Bedeutung wie in Formel I besitzen, dadurch gekennzeichnet, daß man die Dehydrierung mit $H_2O_2$ und in zwei Stufen durchführt, indem man

a) die Dihydrothiophene der Formeln IIa und/oder IIb mit einer mindestens etwa äquimolaren Menge $H_2O_2$ in einem neutralen Lösungs- und Verdünnungsmittel in Abwesenheit von Säure(n) umsetzt, wobei die 1-Oxide der Ausgangs-Dihydrothiophene entstehen und

b) die in Stufe a) gebildeten Dihydrothiophen-1-oxide ohne oder auch nach ihrer Isolierung mit einer Säure versetzt, wobei sich die Dihydrothiophen-1-oxide in die Thiophenderivate der Formel I umlagern.

**2.** Verfahren zur Herstellung von Dihydrothiophen-1-oxiden der Formeln IIIa und IIIb

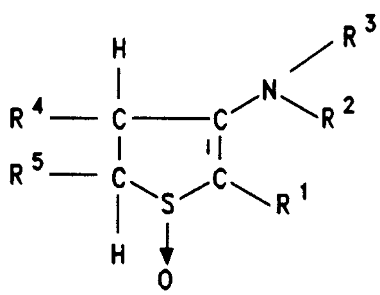

III a

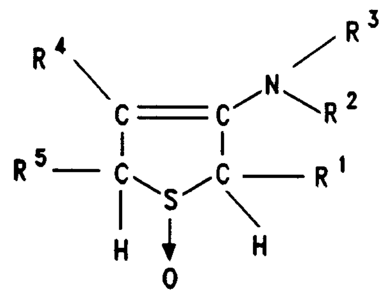

III b

worin $R^1$ bis $R^5$ unabhängig voneinander die in Anspruch 1 genannte Bedeutung haben, dadurch gekennzeichnet, daß man Dihydrothiophene der Formeln IIa bzw. IIb

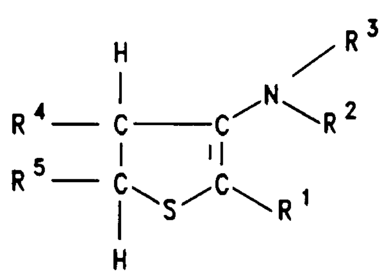

II a

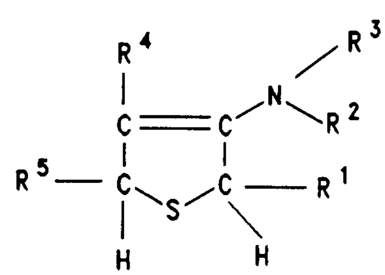

II b

worin $R^1$ bis $R^5$ die gleiche Bedeutung wie in den Formeln IIIa und IIIb besitzen, mit einer mindestens

etwa äquimolaren Menge $H_2O_2$ in einem neutralen Lösungs- oder Verdünnungsmittel in Abwesenheit von Säure(n) umsetzt.

3. Verfahren zur Herstellung von Thiophenderivaten der Formel I gemäß der Definition in Anspruch 1, dadurch gekennzeichnet, daß man Dihydrothiophen-1-oxide der Formeln IIIa und/oder IIIb

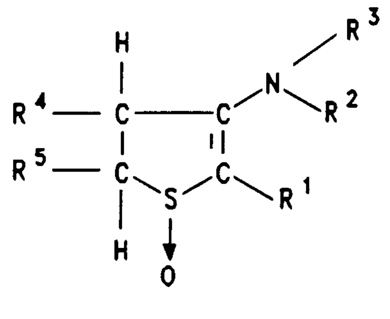

IIIa

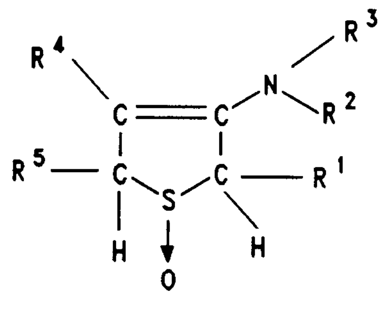

IIIb

worin $R^1$ bis $R^5$ unabhängig voneinander die in Anspruch 1 genannte Bedeutung haben, mit einer Säure versetzt, wobei sich die Dihydrothiophen-1-oxide zu den Thiophenderivaten der Formel I umlagern.

4. Verfahren nach einem oder mehreren der Ansprüche 1 bis 3, dadurch gekennzeichnet, daß man in Wasser oder einem neutralen organischen Lösungsmittel, vorzugsweise in Wasser und/oder einem $C_1$-$C_4$-Alkanol, als Lösungs- oder Verdünnungsmittel arbeitet.

**Claims**

**Claims for the following Contracting States : DE, GB, FR, IT, NL, SE, LI, CH, BE, AT**

1. A process for the preparation of a thiophene derivative of the formula I

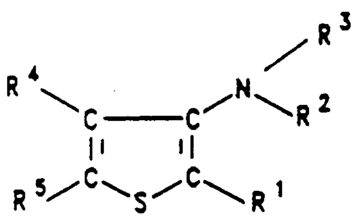

I

in which $R^1$ to $R^5$ have the following meaning:
$R^1$, $R^4$ and $R^5$: independently of one another, H, $C_1$-$C_4$-alkyl, $C_5$-$C_6$-cycloalkyl, $CH_2$-$C_6H_5$, $C_6H_5$, $COOCH_3$, $COOC_2H_5$, $CONH_2$, $COCH_3$, $COC_2H_5$ or CN;
$R^2$ and $R^3$: independently of one another H, $C_1$-$C_4$-alkyl, optionally substituted by $COOCH_3$ or $COOC_2H_5$, $C_5$-$C_6$-cycloalkyl, $(CH_2)_{1-3}$-$C_6H_5$, $C_6H_5$, optionally substituted by OH, $OCH_3$, $OC_2H_5$, F, Cl or Br, or CO($C_1$-$C_5$-alkyl) in which the alkyl radical can be substituted by OH, F, Cl, Br, ($C_1$-$C_4$)-alkoxy, $NH_2$ or ($C_1$-$C_8$)-alkylamino groups, and one of the radicals $R^2$ or $R^3$ can, in addition, also be $COOCH_3$ or $COOC_2H_5$, or $R^2$ and $R^3$, together with the N atom to which they are attached, form a pyrrolidino, piperidino, morpholino, piperazino or homopiperazino ring, by dehydrogenating a dihydrothiophene of the formulae IIa and/or IIb

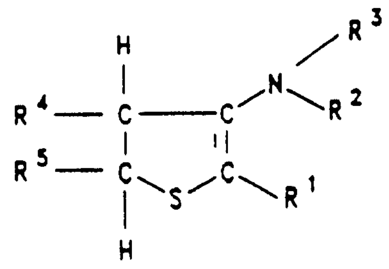

IIa

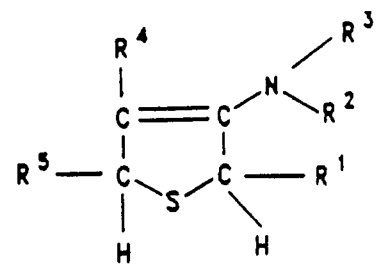

IIb

in which the radicals $R^1$ to $R^5$ have the same meaning as in formula I, which comprises carrying out the dehydrogenation by means of $H_2O_2$ and in two stages,

a) by reacting a dihydrothiophene of the formulae IIa and/or IIb with an at least approximately equimolar amount of $H_2O_2$ in a neutral solvent or diluent in the absence of acid(s), in the course of which the 1-oxide of the initial dihydrothiophene is formed, and

b) by adding an acid to the dihydrothiophene 1-oxide formed in stage a), without or after isolation of the latter, in the course of which the dihydrothiophene 1-oxide undergoes rearrangement into the thiophene derivative of the formula I.

**2.** A process for the preparation of a dihydrothiophene 1-oxide of the formulae IIIa and IIIb

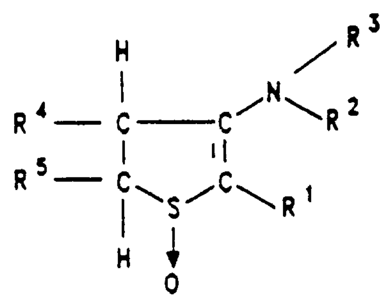

IIIa

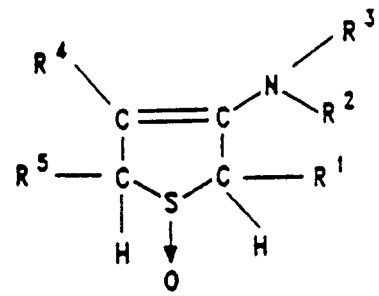

IIIb

in which $R^1$ to $R^5$ independently of one another have the meaning indicated in claim 1, wherein a dihydrothiophene of the formulae IIa or IIb

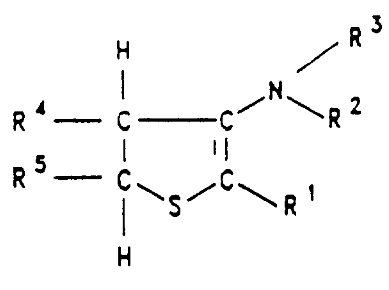

IIa

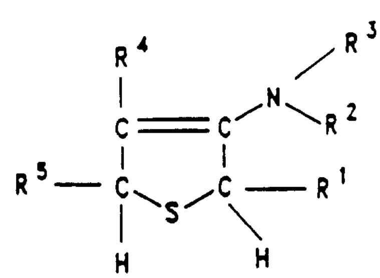

IIb

in which $R^1$ to $R^5$ have the same meaning as in the formulae IIIa and IIIb is reacted with an at least approximately equimolar amount of $H_2O_2$ in a neutral solvent or diluent in the absence of acid(s).

3. The process for the preparation of a thiophene derivative of the formula I as claimed in the definition in claim 1, wherein an acid is added to a dihydrothiophene 1-oxide of the formulae IIIa and/or IIIb

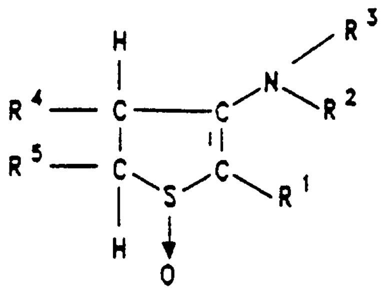

IIIa

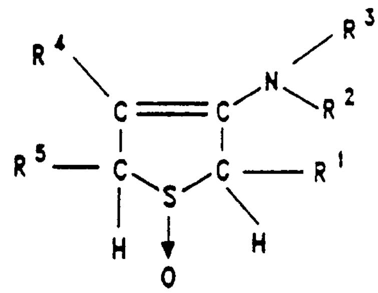

IIIb

in which $R^1$ to $R^5$ independently of one another have the meaning indicated in claim 1, in the course of which the dihydrothiophene 1-oxide undergoes rearrangement to give a thiophene derivative of the formula I.

4. The process as claimed in one or more of claims 1-3, wherein the reaction is carried out in water or a neutral organic solvent, preferably in water and/or a $C_1$-$C_4$-alkanol, as a solvent or diluent.

5. A dihydrothiophene 1-oxide of the formulae IIIa and IIIb

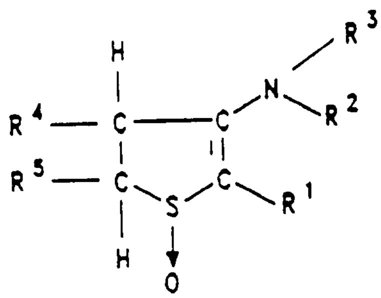

IIIa

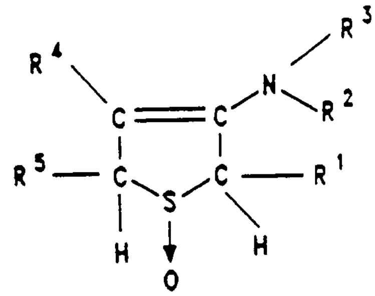

IIIb

in which $R^1$ to $R^5$ have the meaning indicated in claim 1.

**Claims for the following Contracting State : ES**

1. A process for the preparation of a thiophene derivative of the formula I

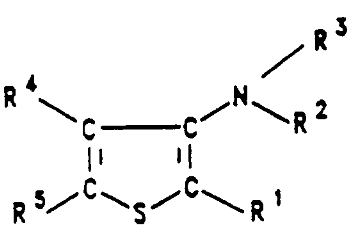

I

in which $R^1$ to $R^5$ have the following meaning:
$R^1$, $R^4$ and $R^5$: independently of one another, H, $C_1$-$C_4$-alkyl, $C_5$-$C_6$-cycloalkyl, $CH_2$-$C_6H_5$, $C_6H_5$, $COOCH_3$, $COOC_2H_5$, $CONH_2$, $COCH_3$, $COC_2H_5$ or CN;
$R^2$ and $R^3$: independently of one another H, $C_1$-$C_4$-alkyl, optionally substituted by $COOCH_3$ or $COOC_2H_5$, $C_5$-$C_6$-cycloalkyl, $(CH_2)_{1-3}$-$C_6H_5$, $C_6H_5$, optionally substituted by OH, $OCH_3$, $OC_2H_5$, F, Cl

or Br, or CO($C_1$-$C_5$-alkyl) in which the alkyl radical can be substituted by OH, F, Cl, Br, ($C_1$-$C_4$)-alkoxy, $NH_2$ or ($C_1$-$C_8$)-alkylamino groups, and one of the radicals $R^2$ or $R^3$ can, in addition, also be $COOCH_3$ or $COOC_2H_5$, or $R^2$ and $R^3$, together with the N atom to which they are attached, form a pyrrolidino, piperidino, morpholino, piperazino or homopiperazino ring, by dehydrogenating a dihydrothiophene of the formulae IIa and/or IIb

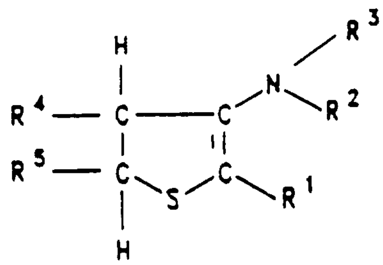

IIa

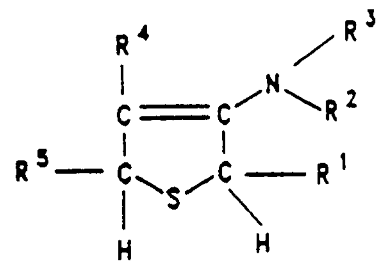

IIb

in which the radicals $R^1$ to $R^5$ have the same meaning as in formula I, which comprises carrying out the dehydrogenation by means of $H_2O_2$ and in two stages,

a) by reacting a dihydrothiophene of the formulae IIa and/or IIb with an at least approximately equimolar amount of $H_2O_2$ in a neutral solvent or diluent in the absence of acid(s), in the course of which the 1-oxide of the initial dihydrothiophene is formed, and

b) by adding an acid to the dihydrothiophene 1-oxide formed in stage a), without or after isolation of the latter, in the course of which the dihydrothiophene 1-oxide undergoes rearrangement into the thiophene derivative of the formula I.

**2.** A process for the preparation of a dihydrothiophene 1-oxide of the formulae IIIa and IIIb

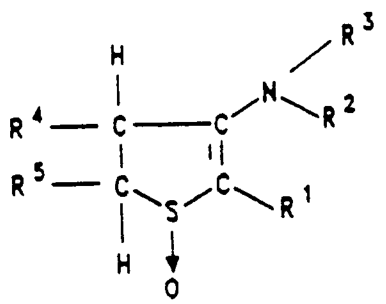

IIIa

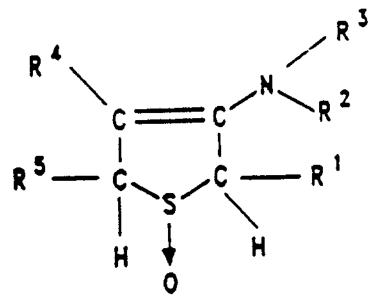

IIIb

in which $R^1$ to $R^5$ independently of one another have the meaning indicated in claim 1, wherein a dihydrothiophene of the formulae IIa or IIb

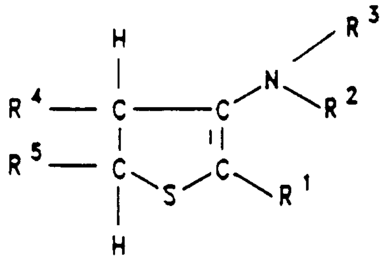

IIa

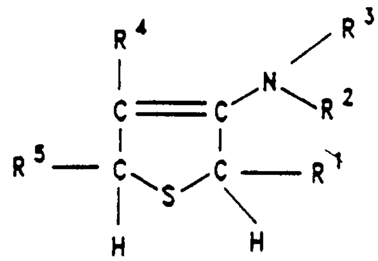

IIb

in which $R^1$ to $R^5$ have the same meaning as in the formulae IIIa and IIIb is reacted with an at least approximately equimolar amount of $H_2O_2$ in a neutral solvent or diluent in the absence of acid(s).

**3.** The process for the preparation of a thiophene derivative of the formula I as claimed in the definition in claim 1, wherein an acid is added to a dihydrothiophene 1-oxide of the formulae IIIa and/or IIIb

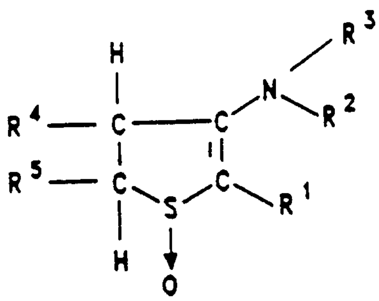

IIIa

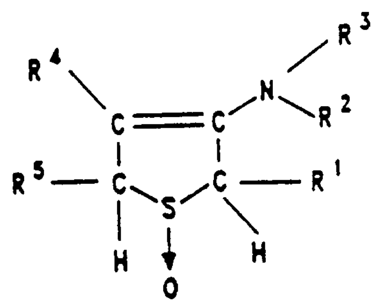

IIIb

in which $R^1$ to $R^5$ independently of one another have the meaning indicated in claim 1, in the course of which the dihydrothiophene 1-oxide undergoes rearrangement to give a thiophene derivative of the formula I.

**4.** The process as claimed in one or more of claims 1-3, wherein the reaction is carried out in water or a neutral organic solvent, preferably in water and/or a $C_1$-$C_4$-alkanol, as a solvent or diluent.

**Revendications**
**Revendications pour les Etats contractants suivants : DE, GB, FR, IT, NL, SE, LI, CH, BE, AT**

**1.** Procédé pour préparer des dérivés du thiophène de formule I

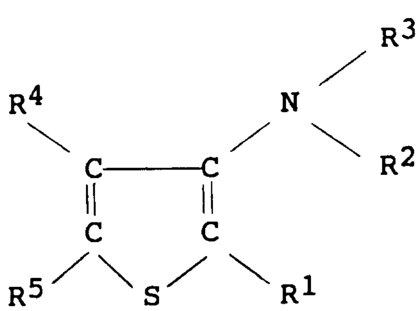

I

dans laquelle les radicaux $R^1$ à $R^5$ présentent la signification suivante :

$R^1$, $R^4$ et $R^5$ : indépendamment les uns des autres, H,

alkyle en $C_1$ à $C_4$,
cycloalkyle en $C_5$ à $C_6$,
$CH_2$-$C_6H_5$,
$C_6H_5$,
$COOCH_3$,
$COOC_2H_5$,
$CONH_2$,
$COCH_3$,
$COC_2H_5$ ou
CN ;

$R^2$ et $R^3$ : indépendamment l'un de l'autre, H, alkyle en $C_1$ à $C_4$, éventuellement substitué par $COOCH_3$ ou $COOC_2H_5$,
cycloalkyle en $C_5$ à $C_6$,
$C_6H_5$-$(CH_2)_{1\ à\ 3}$,
$C_6H_5$, éventuellement substitué par OH, $OCH_3$, $OC_2H_5$, F, Cl ou Br,
CO(alkyle en $C_1$ à $C_5$), où le radical alkyle peut être substitué par OH, F, Cl, Br, oxy(alkyle en $C_1$ à $C_4$), $NH_2$ ou des groupes amino(alkyle en $C_1$ à $C_8$),

et l'un des radicaux $R^2$ ou $R^3$ peut être en outre $COOCH_3$ ou $COOC_2H_5$,

ou $R^2$ et $R^3$ forment, conjointement avec l'atome d'azote auquel ils sont liés, un cycle de type pyrrolidine, pipéridine, morpholine, pipérazine ou homopipérazine, par déshydrogénation de dihydrothiophènes de formules IIa et/ou IIb

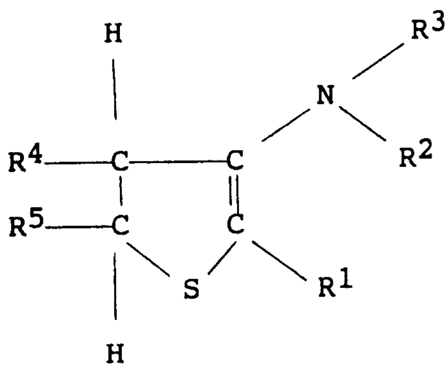

IIa

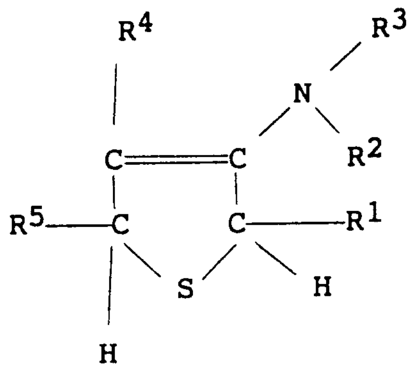

IIb

où les radicaux $R^1$ à $R^5$ ont la même signification que dans la formule I, caractérisé en ce que l'on effectue la déshydrogénation avec $H_2O_2$ et en deux étapes consistant à :

a) faire réagir les dihydrothiophènes de formules IIa et/ou IIb avec une quantité au moins environ équimolaire de $H_2O_2$, dans un solvant ou un agent de dilution neutre, en l'absence d'acide(s), ce qui permet de former les oxydes en position 1 des dihydrothiophènes de départ, et

b) mélanger les oxydes de dihydrothiophène-1 formés dans l'étape a), sans les isoler ou après les avoir isolés, avec un acide, ce qui permet de réarranger les oxydes de dihydrothiophène-1 en dérivés du thiophène de formule I.

**2.** Procédé pour préparer des oxydes de dihydrothiophène-1 de formules IIIa et IIIb

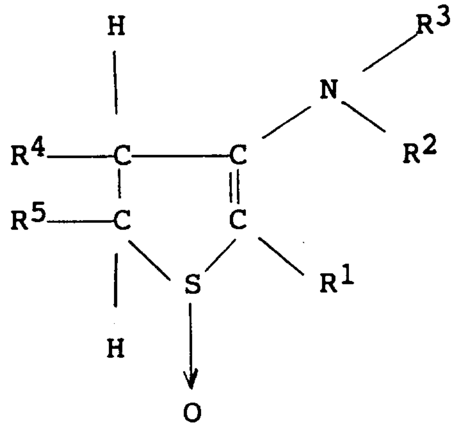

IIIa

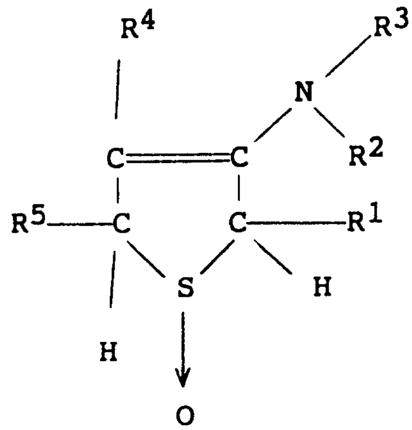

IIIb

où les radicaux $R^1$ à $R^5$ présentent, indépendamment les uns des autres, la signification citée dans la revendication 1, caractérisé en ce que l'on fait réagir des dihydrothiophènes de formules IIa ou IIb

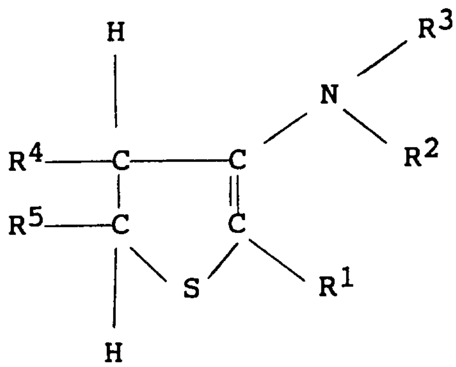

IIa

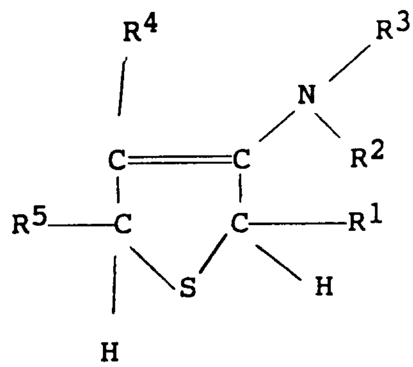

IIb

où les radicaux $R^1$ à $R^5$ ont la même signification que dans les formules IIIa et IIIb, avec une quantité au moins environ équimolaire de $H_2O_2$, dans un solvant ou un agent de dilution neutre, en l'absence d'acide(s).

**3.** Procédé pour préparer des dérivés du thiophène de formule I selon la définition de la revendication 1, caractérisé en ce que l'on mélange des oxydes de dihydrothiophène-1 de formules IIIa et/ou IIIb

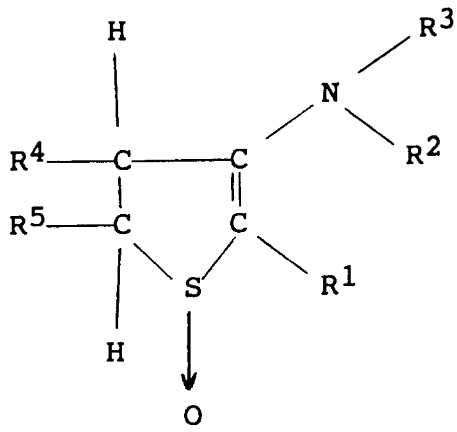

IIIa

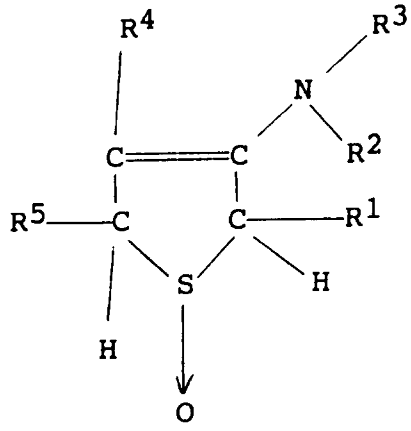

IIIb

où les radicaux $R^1$ à $R^5$ présentent, indépendamment les uns des autres, la signification citée dans la revendication 1, avec un acide, ce qui permet de réarranger les oxydes de dihydrothiophène-1 en dérivés du thiophène de formule I.

**4.** Procédé selon une ou plusieurs des revendications 1 à 3, caractérisé en ce que l'on travaille dans l'eau ou dans un solvant organique neutre, de préférence dans l'eau et/ou dans un alcool en $C_1$ à $C_4$, en tant que solvant ou agent de dilution.

**5.** Oxydes de dihydrothiophène-1 de formules IIIa et IIIb

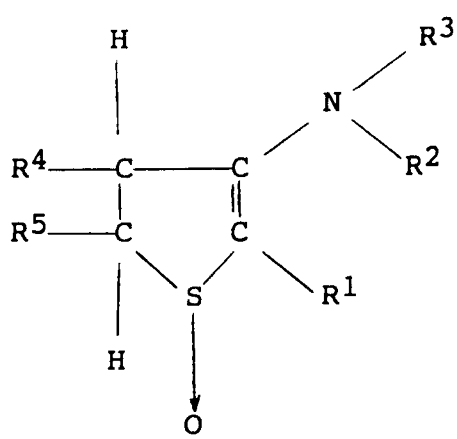

IIIa

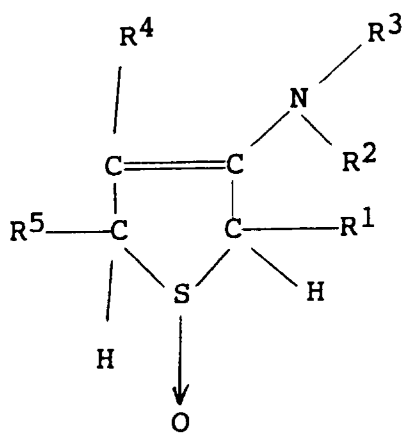

IIIb

où les radicaux $R^1$ à $R^5$ ont la signification citée dans la revendication 1.

**Revendications pour l'Etat contractant suivants : ES**

1. Procédé pour préparer des dérivés du thiophène de formule I

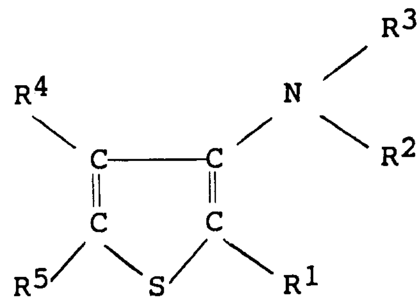

I

dans laquelle les radicaux $R^1$ à $R^5$ présentent la signification suivante :

$R^1$, $R^4$ et $R^5$ : indépendamment les uns des autres, H, alkyle en $C_1$ à $C_4$, cycloalkyle en $C_5$ à $C_6$, $CH_2$-$C_6H_5$, $C_6H_5$, $COOCH_3$, $COOC_2H_5$, $CONH_2$, $COCH_3$, $COC_2H_5$ ou CN ;

$R^2$ et $R^3$ : indépendamment l'un de l'autre, H, alkyle en $C_1$ à $C_4$, éventuellement substitué par $COOCH_3$ ou $COOC_2H_5$, cycloalkyle en $C_5$ à $C_6$, $C_6H_5$-$(CH_2)_{1\ à\ 3}$, $C_6H_5$, éventuellement substitué par OH, $OCH_3$, $OC_2H_5$, F, Cl ou Br, CO(alkyle en $C_1$ à $C_5$), où le radical alkyle peut être substitué par OH, F, Cl, Br, oxy(alkyle en $C_1$ à $C_4$), $NH_2$ ou des groupes amino(alkyle en $C_1$ à $C_8$),

et l'un des radicaux $R^2$ ou $R^3$ peut être en outre $COOCH_3$ ou $COOC_2H_5$, ou $R^2$ et $R^3$ forment, conjointement avec l'atome d'azote auquel ils sont liés, un cycle de type pyrrolidine, pipéridine, morpholine, pipérazine ou homopipérazine, par déshydrogénation de dihydrothiophènes de formules IIa et/ou IIb

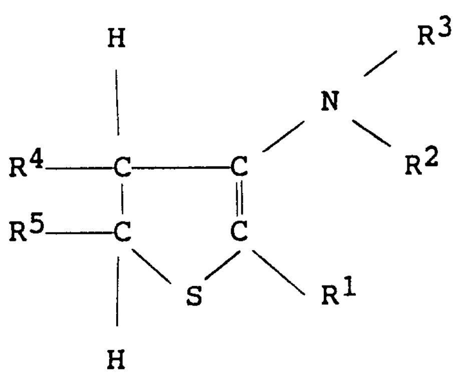

IIa

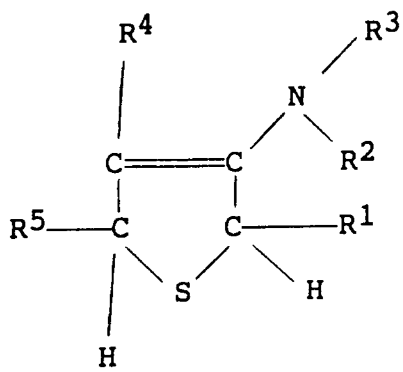

IIb

où les radicaux $R^1$ à $R^5$ ont la même signification que dans la formule I, caractérisé en ce que l'on effectue la déshydrogénation avec $H_2O_2$ et en deux étapes consistant à :

a) faire réagir les dihydrothiophènes de formules IIa et/ou IIb avec une quantité au moins environ équimolaire de $H_2O_2$, dans un solvant ou un agent de dilution neutre, en l'absence d'acide(s), ce qui permet de former les oxydes en position 1 des dihydrothiophènes de départ, et

b) mélanger les oxydes de dihydrothiophène-1 formés dans l'étape a), sans les isoler ou après les avoir isolés, avec un acide, ce qui permet de réarranger les oxydes de dihydrothiophène-1 en dérivés du thiophène de formule I.

**2.** Procédé pour préparer des oxydes de dihydrothiophène-1 de formules IIIa et IIIb

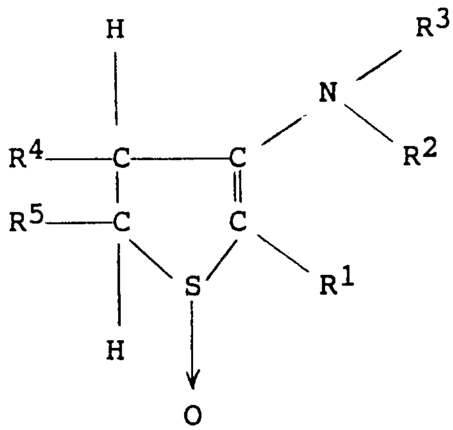

IIIa

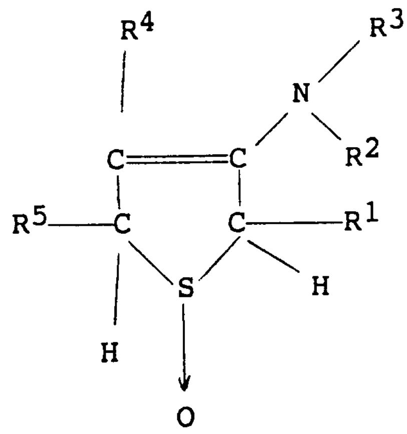

IIIb

où les radicaux $R^1$ à $R^5$ présentent, indépendamment les uns des autres, la signification citée dans la revendication 1, caractérisé en ce que l'on fait réagir des dihydrothiophènes de formules IIa ou IIb

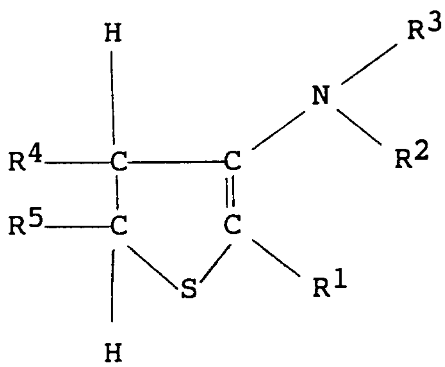

IIa

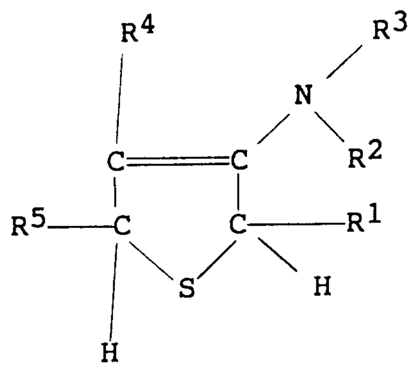

IIb

où les radicaux $R^1$ à $R^5$ ont la même signification que dans les formules IIIa et IIIb, avec une quantité au moins environ équimolaire de $H_2O_2$, dans un solvant ou un agent de dilution neutre, en l'absence d'acide(s).

**3.** Procédé pour préparer des dérivés du thiophène de formule I selon la définition de la revendication 1, caractérisé en ce que l'on mélange des oxydes de dihydrothiophène-1 de formules IIIa et/ou IIIb

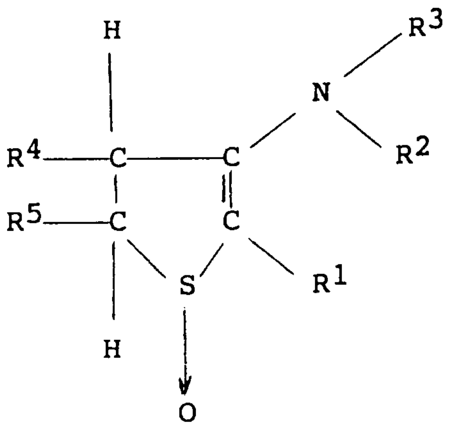

IIIa

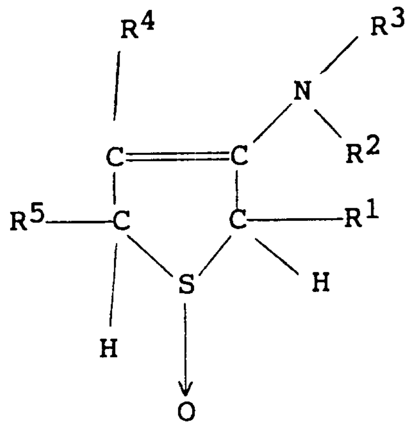

IIIb

où les radicaux $R^1$ à $R^5$ présentent, indépendamment les uns des autres, la signification citée dans la revendication 1, avec un acide, ce qui permet de réarranger les oxydes de dihydrothiophène-1 en dérivés du thiophène de formule I.

**4.** Procédé selon une ou plusieurs des revendications 1 à 3, caractérisé en ce que l'on travaille dans l'eau ou dans un solvant organique neutre, de préférence dans l'eau et/ou dans un alcool en $C_1$ à $C_4$, en tant que solvant ou agent de dilution.